# EUROPEAN PATENT SPECIFICATION

(11) **EP 2 747 762 B1**
(45) Date of publication and mention of the grant of the patent: **01.07.2015**
(21) Application number: 12756594.3
(22) Date of filing: 23.08.2012
(51) Int. Cl.: A61K 31/381, A61P 27/02

(54) **USE OF ZILEUTON FOR THE TREATMENT OF NASAL POLYPS IN CYSTIC FIBROSIS PATIENTS**
VERWENDUNG VON ZILEUTON ZUR BEHANDLUNG VON NASENPOLYPEN BEI PATIENTEN MIT ZYSTISCHER FIBROSE
UTILISATION DE ZILEUTON DANS LE TRAITEMENT DES POLYPES DU NEZ CHEZ DES PATIENTS ATTEINTS DE LA MUCOVISCIDOSE

(30) Priority: 23.08.2011 US 201161526440 P
(43) Date of publication of application: 02.07.2014
(60) Divisional application: 15168972.6
(73) Proprietor: Cornerstone Therapeutics Inc., Cary, North Carolina 27518 (US); Han, Joseph, K., Virginia Beach, VA 23454 (US)
(72) Inventor: HAN, Joseph, K., Virginia Beach, VA 23454 (US); DELL'ANNA, Carmen, Cary, NC 27518 (US)
(74) Representative: Potter Clarkson LLP
(86) International application number: PCT/US2012/052094
(87) International publication number: WO 2013/028880

(56) References cited:
- WO-A2-2007/098189
- US-A- 4 873 259
- CLAEYS S ET AL: "Nasal polyps in patients with and without cystic fibrosis: a differentiation by innate markers and inflammatory mediators.", CLINICAL AND EXPERIMENTAL ALLERGY : JOURNAL OF THE BRITISH SOCIETY FOR ALLERGY AND CLINICAL IMMUNOLOGY APR 2005 LNKD- PUBMED:15836755, vol. 35, no. 4, April 2005 (2005-04), pages 467-472, XP002685304, ISSN: 0954-7894
- FOKKENS ET AL.: RHINOLOGY, vol. 45, no. 20, 16 October 2007 (2007-10-16), pages 1-139, XP008091259, cited in the application
- HENRIKSSON ET AL.: CHEST, vol. 121, 2002, pages 40-47, XP002685306, cited in the application
- PARNES S M ET AL: "Acute effects of antileukotrienes on sinonasal polyposis and sinusitis.", EAR, NOSE, & THROAT JOURNAL JAN 2000 LNKD- PUBMED:10665187, vol. 79, no. 1, January 2000 (2000-01), pages 18-20 , 24, XP9163737, ISSN: 0145-5613

## Description

### Background of the Invention

Cystic fibrosis (CF) is the most common autosomal recessive condition resulting in morbidity and mortality among Caucasians, affecting about 30,000 children and adults in the United States. Adult CF patients (18 years and older) have been estimated to number about 47% of this total in 2009, or approximately 14,000 individuals (Taniguchi et al, Allergology International, 57:313-320, 2008 and Cystic Fibrosis Foundation website). Cystic fibrosis is caused by mutations in the CF transmembrane conductance regulator gene (CFTR) which encodes for a protein that functions as a cyclic adenosine monophosphate-regulated chloride channel (Henriksson et al., Chest, 121:40-47, 2002). Abnormal function of this protein results in aberrant conductance across the apical membrane of the epithelial cells of the lung, pancreas, sweat glands, liver, salivary glands, colon and nasal mucosa (Henriksson et al, 2002). The resultant dysfunction of the mucosal interface of the upper respiratory tract is clinically manifest as frequent otorhinolaryngological manifestations of CF, including chronic rhinosinusitis and nasosinusal polyposis (Claeys et al, Clin. Exp. Allergy, 35:467-472, 2005). Although these conditions do not result in mortality, they cause considerable morbidity and negatively impact the quality of life of many CF patients due to symptomatology (Pimenta et al, Intl. Arch. Otorhinolaryngol., 12:552-558, 2008).

Nasal polyps are polypoidal masses arising mainly as overgrowths from the mucous membranes of the nose and paranasal sinuses and are often freely movable and nontender. Antrochoanal polyps arise from the maxillary sinuses and are typically single and unilateral, while ethmoidal polyps arise from the ethmoidal sinuses and are typically multiple and bilateral. Compared to the general population, the prevalence of nasal polyps (NPs) is considerably higher in CF patients (Table 1), ranging from 32 to 56% of patients (Henriksson et al, 2002; Coste et al, Rhinology, 33:152-156, 1995;

Kerrebijn et al., Eur. Respir. J., 5:1239-42, 1992; Brihaye et al., Int. J. Pediatr. Otorhinolaryngol., 28(2-3):141-147, 1994; Hadfield et al., Clin. Otolaryngol Allied Sci., 25:19-22, 2000; DeGaudemar et al., Rhinology, 34:194-197, 1996; Jorissen et al., Am. J. Respir. Crit. Care Med., 159:1412-1416, 1999; Sakano et al., Int. J. Pediatr. Otorhinolaryngol, 71:41-50, 2007).

**Table 1: Literature reporting prevalence of nasal polyps in patients with CF**

| MEAN AGE (yrs) | AGE RANGE (yrs) | PREVALENCE (%) | REFERENCE |
|---|---|---|---|
| 13.5 | 13 months-31 yrs | 44 | Coste *et al.,* 1995 |
| 26 | 17-40 | 44 | Kerrebijn *et al.,* 1992 |
| 15 | 5-34 | 45 | Brihaye *et al.,* 1994 |
| 28 | 16-58 | 37 | Hadfield *et al.,* 2000 |
| 10 | 1-23 | 32 | DeGaudemar *et al.,* 1996 |
| 18±8 | 2-37 | 39 | Henriksson et al, 2002 |
| 11.6±6.6 (overall CF population) | 1-28 | 56 | Jorissen *et al.,* 1999 |
| N/A | <2-15yrs | 36 | Sekano *et al.,* 2007 |

Although polyps are commonly reported in CF children aged 5-14 years, they can also develop in older CF patients (Sheahan, RJ. Harvey and RJ Schlosser, Nasal polyps in Cystic Fibrosis. Book chapter Nasal Polyps: pathogenesis, medical and surgical treatment. 2010: 145-152*;* Kerrebijn et al., Eur Respir J. 1992;5:1239-42*;* Hadfield et al., Clin Otolaryngol Allied Sci. 2000; 25:19-22*;* and Becker et al., Am J Rhinol 2007; 21:478-482*),* and the prevalence of nasosinusal polyposis in the adult and adolescent CF population has been described to be as high as 44-45% (Kerrebijn *et al,* 1992 and Brihaye et al., Int J Pediatr Otorhinolaryngol. 1994 Jan; 28(2-3):141-7*).* Therefore, it is estimated that approximately 6,000-7,000 adults and children 12 years of age and older in the US have nasal polyps complicating CF.

In patients with CF, intracellular water flux increases mucous viscosity, leading to mucociliary dysfunction, stasis, and nasosinusal obstruction (Batsakis and E1-Naggar, Ann. Otol. Rhinol. Laryngol., 105:329-330, 1996). This predisposes the CF patient to bacterial colonization, mainly by *Pseudomonas* and *Staphylococcus* species, and results in chronic infection. However, the current cause of nasal polyps in cystic fibrosis patients is unknown.

The symptoms of nasosinusal polyposis in CF include nasal obstruction, nasal congestion, rhinorrhea, sinusitis, cough, headache, facial pain, disordered sleep, anosmia and secondary infection (Pimenta *et al.,* 2008; Hadfield *et al.,* 2000; Sheahan *et al.,* 2010; and Gysin et al., Pediatr Pulmonol 2000; 30:481-489*).* These symptoms are often underestimated due to the priority given to the more severe manifestations of cystic fibrosis, such as pulmonary infections and nutritional impairment (Pimenta *et al.,* 2008). Because patients may adapt to their nasosinusal symptoms, these may go unreported and thus untreated, despite the fact that otorhinolaryngological manifestations of the disease can interfere with quality of life and may have a role in the overall progression of CF (Piment *et al.,* 2008). There is some evidence of a correlation between upper airway abnormalities and lung disease in CF patients (Friedman and Stewart, Am. J. Rhinol., 20:568-72, 2006), and the paranasal sinuses may serve as a source for *Pseudomonas aeruginosa-*induced lung infections in CF (Fokkens et al., Rhinology 45; suppl. 20: 1-139). This association between *Pseudomonas* respiratory colonization and the presence of nasal polyposis may contribute to the higher annual rates of cystic fibrosis-related acute exacerbations and hospitalizations observed in some clinical trials in CF patients with polyposis in comparison to CF patients without polyposis (Cimmino et al., Clin Otolaryngol 2003; 28:125-132*).*

Current medical treatments employed in nasosinusal polyposis in CF include topical nasal steroids, oral steroids, nasal irrigations, antibiotics, decongestants, and mucolytics (Sheahan *et al.,* 2010). In advanced cases, surgical debridement may also be required to remove the polyps and restore sinus ventilation. However, these treatments are often unsatisfactory.

Topical intranasal corticosteroids have been used as long-term monotherapy in mild cases or in combination with systemic corticosteroids and/or surgery in more severe cases (Mygind and Lund, Treat Respir Med. 2006; 5:93-102*).* When CF polyps are small, use of topical intranasal steroids usually results in temporary polyp shrinkage and reduction of the associated symptoms (Henriksson *et al.,* 2002). However, steroids block inflammation at a high level and typically temporarily reduce, but do not eliminate, these nasal polyps in CF patients. Moreover, many CF patients with polyposis commonly do not respond to steroids at all (McClay, Nasal Polyps, E-medicine specialties review, Updated: Oct. 22, 2008. http://emedicine.medscape.com/article/994274-treatment; and Scadding, Curr Allergy Asthma Rep. 2002;2:494-9). Indeed, in the only prospective trial available in the literature that evaluated topical corticosteroid treatment for nasal polyps in adult patients with CF, no significant improvement in symptoms was observed in the steroid-treated group compared with placebo treatment (Hadfield et al., Rhinology. 2000;38:63-5). The historically poor symptomatic response to steroids may be one of the reasons why clinical development programs that have evaluated topical steroids in nasal polyposis (*i.e.,* mometasone furoate, beclomethasone diproprionate) excluded patients with CF from the studied populations (Pär Stjärne et al., Arch Otolaryngol Head Neck Surg. 2006;132:179-185 and Small et al., Allergy Clin Immunol 2005;116:1275-81).

Administration of steroids also has unwanted and potentially severe side effects. When administered intranasally, steroids can cause thinning of the nasal mucosa and subsequent bleeding. When administered systemically for long periods of time, steroids may have severe side effects, including elevation of blood pressure, insomnia, agitation, psychosis, increased susceptibility to infection, easy bruising, weight gain, osteoporosis and joint damage, hyperglycemia and worsening diabetes, cataracts, and muscular weakness.

Since nasal polyps in CF patients are commonly non-responsive to standard steroid therapy, surgical debridement is often necessary (Gysin *et al.,* 2000). Indeed, polyp surgery is the second most common class of operations performed in CF patients (Henriksson *et al.,* 2002). However, due to a high tendency for polyps to recur (about 60% of patients have symptomatic recurrence within 18 months following polypectomy), repeated sinus surgery (including endoscopic surgery) is often needed to provide symptomatic relief (Henriksson *et al.,* 2002). Moreover, debridement risks include injury to the eye or brain, spinal fluid leak, loss of sense of smell and nosebleeds.

Thus, no anti-inflammatory drugs currently approved for the treatment of nasal polyps have been successful in treating patients with nasosinusal polyps complicating cystic fibrosis. Accordingly, nasal polyps in cystic fibrosis patients are a problem of major clinical concern for which effective treatments are currently lacking.

### Summary of the Invention

It has now surprisingly been found that zileuton ((±)-1-(1-Benzo[b]thien-2-ylethyl)-1-hydroxyurea), an inhibitor of 5-lipoxygenase, can be used to effectively treat nasal polyps in patients having cystic fibrosis. Zileuton has the following chemical structure:

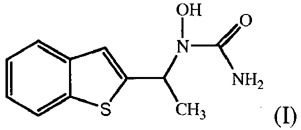

According to the present invention, there is provided zileuton ((±)-1-(1-Benzo[b]thien-2-ylethyl)-1-hydroxyurea) for use in the treatment of nasal polyps in a patient having cystic fibrosis. In one embodiment, the patient has previously been treated with a steroid.

In another aspect, the invention provides zileuton for use in the treatment of nasal polyps in a patient having cystic fibrosis wherein the patient is also administered a steroid. In one embodiment, the steroid is administered to the patient for a period of time sufficient to reduce the size of the nasal polyps by at least 50%. In another embodiment, the administration of zileuton is begun after the 50% reduction in size of the nasal polyps. In another embodiment, the administration of the steroid and zileuton is concurrent, and administration of zileuton is continued after the 50% reduction in size of the nasal polyps for a period of time sufficient to maintain the size reduction of the polyps. In yet another embodiment, the steroid is administered for a period of time sufficient to reduce the size of the nasal polyps by at least 75%, and administration of zileuton is begun after the 75% reduction in size of the nasal polyps. In another embodiment, the steroid is administered for a period of time sufficient to reduce the size of the nasal polyps by at least 90%, and administration of zileuton is begun after the 90% reduction in size of the nasal polyps.

In another aspect, the invention provides administration of zileuton is begun after the nasal polyps have been debridged. In one embodiment, the patient is also administered a steriod. In another embodiment, the zileuton is administered for a period of time sufficient to reduce the risk of recurrence of nasal polyps.

In one embodiment, zileuton is substantially free of (S)-zileuton. Also described herein, zileuton is administered at a dose of about 450 milligrams to about 2400 milligrams per day. Alternatively, zileuton is administered at a dose of about 600 milligrams per day. In yet another example, zileuton is administered at a dose of about 1200 milligrams per day. In another example, zileuton is administered at a dose of about 2400 milligrams per day.

In one example, zileuton is administered orally. In another example, zileuton is administered as a single daily dose. In another example, zileuton is administered twice per day. In another example, zileuton is administered for at least four weeks.

In one example, zileuton comprises zileuton that is at least 70% by weight (R)-zileuton and 10% by weight or less of (S)-zileuton, wherein said percent is based on the total weight of zileuton administered.

In one embodiment, the steroid is a steroid nasal spray. In another embodiment, the steroid nasal spray is fluticasone, budesonide, flunisolide, mometasone,
triamcinolone, or beclomethasone.

In another embodiment, the steroid is an oral steroid. In one embodiment, the oral steroid is prednisolone, prednisone, methylprednisolone or dexamethasone.

In another aspect, the invention provides zileuton for use in reducing the likelihood of developing nasal polyps in a patient having cystic fibrosis and a *Pseudomonas* infection.

Also described herein is a method of reducing the likelihood of developing nasal polyps in a patient having cystic fibrosis comprising testing the patient for a *Pseudomonas* infection and, wherein the patient has a *Pseudomonas* infection, administering a therapeutically effective amount of zileuton to the patient for a period of time sufficient to reduce the likelihood of development of nasal polyps.

### Detailed Description of the Invention

The present invention provides zileuton for use in the treatment of nasal polyps in a patient having cystic fibrosis according to Claim 1. The invention is based, at least in part, on the surprising discovery that zileuton can be used to decrease the size of nasal polyps, number of nasal polyps, and/or severity of symptoms of nasal polyps in a patient having cystic fibrosis without the unwanted side effects and high rates of reoccurrence associated with current medical treatments, such as steroids and surgical debridement.

The method of treating cystic fibrosis patients with nasal polyps includes a monotherapy, *e.g.,* wherein zileuton is the only pharmaceutically active agent being used to treat the nasal polyps. This embodiment consists essentially of administering an effective amount of zileuton to the patient. "Consisting essentially of" in this context excludes steroid administration and/or surgical intervention, but includes other medical treatments, such as administering other pharmaceutically active agents commonly used in the management of systems of cystic fibrosis other than nasal polyps. In one embodiment, the patient has previously been treated with steroids, and the steroid treatment was ineffective. In another embodiment, the nasal polyps are characterized by the presence of neutrophils.

Zileuton is administered for a period of time sufficient to reduce the size of the nasal polyps and/or to reduce the number of the nasal polyps by at least 50%, 55%, 60%, 65%, 70%, 75%, 80%, 85%, 90%, 95%, 96%, 97%, 98%, or 99%, or for a period of time sufficient to reduce the size and/or number of nasal polyps below the limits of detection. The size and/or number of nasal polyps can be assessed using methods well known to one of ordinary skill in the art. As an example, nasal polyps can be graded by size and number in both the left and right nasal fossa on a scale of 0 to 3 (0= no polyps; 1 = polyp in the middle meatus, not reaching below the inferior border of the middle turbinate; 2 = polyp reaching below the inferior border of the middle turbinate but no the inferior border of the inferior turbinate; and 3 = large polyp reaching to or below the lower border of the inferior turbinate or polyps medial to the middle turbinate; the sum of the left and right nasal fossa polyp scores giving the total bilateral polyp grade) as described by Small et al., J. Allergy Clin. Immunol., 116(6):1275-1281, 2005.

In another embodiment, zileuton is administered for a period of time sufficient to decrease the severity of symptoms of the nasal polyps by at least 50%, 55%, 60%, 65%, 70%, 75%, 80%, 85%, 90%, 95%, 96%, 97%, 98%, 99% or 100%. Nasal polyp symptoms, such as nasal congestion or obstruction, loss of sense of smell, anterior rhinorrhea, and postnasal drip, can be assessed using methods well known to one of ordinary skill in the art. As an example, nasal polyp symptoms can be graded on a scale of 0 to 3 (0= none; 1 = mild; 2 = moderate; 3 = severe), or by using peak nasal inspiratory flow (PNIF) as described by Small et al., J. Allergy Clin. Immunol., 116(6):1275-1281, 2005. As another example, nasal polyp symptoms can be assessed using the Nasal Obstruction Symptom Evaluation (NOSE) Scale, as described by Stewart et al., Otolaryngology - Head and Neck Surgery, 130(2):157-163, 2004.

More preferably, zileuton administration is continued for a period of time sufficient to prevent or reduce the likelihood of the reoccurrence of the nasal polyps. Exemplary time periods include one week, two weeks, three weeks, four weeks, five weeks, six weeks, seven weeks, eight weeks, three months, four months, five months, six months, seven months, eight months, nine months, ten months, eleven months, twelve months, thirteen months, fourteen months, fifteen months, sixteen months, seventeen months, eighteen months, one year, two years, three years, or for life. For example, a patient's treatment could be initiated and maintained at a total daily dose of about 2400 mg of zileuton per day for a period of 18 months; or about 900 to about 1200 mg of (R)-zileuton per day for 18 months.

Alternatively, zileuton is administered in combination with surgical debridement. As used herein, "debride" and/or "debriding" refer to the surgical removal of nasal polyp tissue. A nasal polyp can be surgically debrided using a small mechanical suction device or microdebrider. Alternatively, a nasal polyp can be surgically debrided using an endoscope. Debridement is also referred to as "polypectomy" and is normally performed on an outpatient basis. Typically, the nasal polyps are first removed by surgical debridement and zileuton is then administered after surgery to prevent or reduce the likelihood of reoccurrence of the nasal polyps. Time periods after surgery that are sufficient to reduce the likelihood of reoccurrence include one week, two weeks, three weeks, four weeks, five weeks, six weeks, seven weeks, eight weeks, three months, four months, five months, six months, seven months, eight months, nine months, ten months, eleven months, twelve months, thirteen months, fourteen months, fifteen months, sixteen months, seventeen months, eighteen months, one year, two years, three years, or for life.

In another alternative, zileuton administration is combined with steroid therapy, *i.e.,* the patient with the nasal polyps is administered an effective amount of zileuton and an effective amount of a steroid. In one embodiment, the steroid is a steroid nasal spray, such as fluticasone, budesonide, flunisolide, mometasone, triamcinolone, or beclomethasone. In another aspect, the steroid is an oral steroid, such as prednisolone, prednisone, dexamethasone, or methylprednisolone. Typically, zileuton and the steroid are initially administered concurrently, and the steroid therapy is terminated once the size and/or number of nasal polyps have been reduced by at least 50%, 55%, 60%, 65%, 70%, 75%, 80%, 85%, 90%, 95%, 96%, 97%, 98%, 99%, or below detection levels. In another embodiment, zileuton and the steroid are initially administered concurrently, and the steroid therapy is terminated once the symptoms of the nasal polyps have decreased in severity by at least 50%, 55%, 60%, 65%, 70%, 75%, 80%, 85%, 90%, 95%, 96%, 97%, 98%, 99% or 100%. Treatment with zileuton is then continued for a period of time sufficient to maintain the size or symptom reduction, or to prevent or reduce the likelihood of reoccurrence or regrowth. Time periods after termination of steroid administration that are sufficient to maintain the size or symptom reduction, or to reduce the likelihood of reoccurrence or regrowth, include one week, two weeks, three weeks, four weeks, five weeks, six weeks, seven weeks, eight weeks, three months, four months, five months, six months, seven months, eight months, nine months, ten months, eleven months, twelve months, thirteen months, fourteen months, fifteen months, sixteen months, seventeen months, eighteen months, one year, two years, three years, or for life.

In yet another alternative, a therapeutically effective amount of a steroid is administered to the patient. The steroid therapy is terminated once the size and/or number of nasal polyps have been reduced by at least 50%, 55%, 60%, 65%, 70%, 75%, 80%, 85%, 90%, 95%, 96%, 97%, 98%, 99%, or below detection levels. In another embodiment, the steroid therapy is terminated once the symptoms of the nasal polyps have decreased in severity by at least 50%, 55%, 60%, 65%, 70%, 75%, 80%, 85%, 90%, 95%, 96%, 97%, 98%, 99%, or 100%. After this size, number or symptom reduction has been achieved, a therapeutically effective amount of zileuton is administered to the patient to maintain the reduction or to prevent or reduce the likelihood of reoccurrence or regrowth. Time periods after termination of steroid administration that are sufficient to maintain the size, number or symptom reduction or to reduce the likelihood of reoccurrence or regrowth include one week, two weeks, three weeks, four weeks, five weeks, six weeks, seven weeks, eight weeks, three months, four months, five months, six months, seven months, eight months, nine months, ten months, eleven months, twelve months, thirteen months, fourteen months, fifteen months, sixteen months, seventeen months, eighteen months, one year, two years, three years, or for life.

In another alternative, the invention provides zileuton for use in preventing or reducing the likelihood of developing nasal polyps in a patient having cystic fibrosis. In one embodiment, the patient has a *Pseudomonas* infection. Optionally, a patient is tested for a *Pseudomonas* infection and, wherein the patient has a *Pseudomonas* infection, administering a therapeutically effective amount of zileuton to the patient for a period of time to prevent or reduce the likelihood of development of nasal polyps. The patient may be tested for *Pseudomonas* infection by methods known to one of ordinary skill in the art, such as swabbing the nasal passages and culturing the bacteria. In one embodiment, the *Pseudomonas* infection is present in the upper respiratory tract, the lower respiratory tract, or both the upper and lower respiratory tracts. Time periods that are sufficient to prevent or reduce the likelihood of development of nasal polyps include one week, two weeks, three weeks, four weeks, five weeks, six weeks, seven weeks, eight weeks, three months, four months, five months, six months, seven months, eight months, nine months, ten months, eleven months, twelve months, thirteen months, fourteen months, fifteen months, sixteen months, seventeen months, eighteen months, one year, two years, three years, or for life.

Since patients with cystic fibrosis often have chronic *Pseudomonas* infection, in one embodiment, the zileuton is administered for life. In another embodiment, administration of zileuton is administered even after the *Pseudomonas* infection is no longer detectable. In yet another embodiment, the zileuton is administered until the *Pseudomonas* infection is no longer detectable. Optionally, the patient is tested for *Pseudomonas* infection during the course of treatment with zileuton, and treatment with zileuton is terminated once the *Pseudomonas* infection is no longer detectable.

Zileuton has the chemical structure described above in Formula (I) with one asymmetric center. Zileuton exists as a pair of enantiomers referred to herein as (R)-zileuton and (S)-zileuton. The structure of (R)-zileuton or (+)-zileuton is shown below in Formula (II):

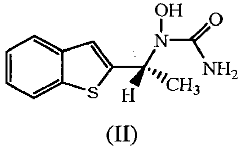

The structure of (S)-zileuton or (-)-zileuton is shown below in Formula (III):

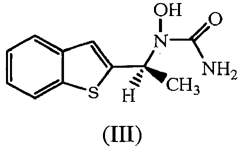

In one embodiment, zileuton used in the methods of the invention is substantially free of (S)-zileuton. The phrases "zileuton substantially free of (S)-zileuton" and "(R)-zileuton substantially free of (S)-zileuton" are used interchangeably herein. In one embodiment, zileuton is substantially free of (S)-zileuton if at least 80% by weight of the zileuton is (R)-zileuton, and 20% or less by weight of the zileuton is (S)-zileuton; or if at least 85%, 90%, 95%, 97%, or 99% by weight of the zileuton is (R)-zileuton and 15%, 10%, 5%, 3%, or 1% or less by weight of the zileuton is (S)-zileuton.

(R)-zileuton may be prepared using chiral synthons or chiral reagents, or resolved using conventional techniques. Methods for the preparation of racemic zileuton have been described, for example, in U.S. Patent Nos. 4,873,259 and 6,080,874 and by Hisao et al, Tetrahedron Letters, 33(19): 2629-32 (1992). (R)-zileuton can be prepared by the resolution of racemic zileuton, such as by using (4S)-4-benzyl-2- oxazolidinone-3-carbonyl chloride (Garigipati et al, Tetrahedron Letters, 34(35): 5537- 40 (1993)). (R)-zileuton can also be chemically resolved using the following:
esterification with oxalyl chloride and R-mandelic acid, isolation of the diastereomeric mixture from cold ethyl acetate, hydrolysis of the diastereomer to yield the (R)-zileuton which can then purified by recrystallization. Methods for the enantioselective synthesis of (R) - zileuton have also been described. For example, a method for the preparation of (R)-zileuton using the addition of Grignard reagents to N-glycosyl nitrones has been described (Basha et al, J Org. Chem., 59(20), 6103-6 (1994)). The enantioselective synthesis of (R)-zileuton has also been described using either L-(+) -lactic acid or a gulofuranose auxiliary (Hsiao et al, 33: 2629-32 (1992); Roloff et al, 35(7): 1011-14 (1994)). Furthermore, a method for the preparation of (R)-zileuton is also described in U.S. Patent No. 5,663,368.

A patient is a human having cystic fibrosis (CF). In one embodiment, the patient is an adult having CF. In yet another embodiment, the patient is an adult under 65 years of age having CF. In another embodiment, the patient is a child under the age of 18 having CF. In another embodiment, the patient is a child under the age of 12 having CF.

An "effective amount" or "therapeutically effective amount" of zileuton and/or a steroid preferably is an amount which, when administered to a patient, results in a decrease in size and/or number of nasal polyps, results in a decrease in severity of nasal polyp symptoms, results in an increase in frequency and duration of nasal polyp symptom-free periods, or reduces the likelihood of impairment or disability due to the nasal polyps. Actual amounts may be varied so as to obtain an amount of the zileuton and/or steroid which is effective to achieve the desired therapeutic response for a particular patient, composition, and mode of administration, without being toxic to the patient. The selected amount will depend upon a variety of pharmacokinetic factors including the activity of the zileuton or the activity of the steroid, the route of administration, the time of administration, the rate of excretion of the zileuton or the steroid, the duration of the treatment, other drugs, compounds and/or materials used in combination with the particular compositions employed, the age, sex, weight, condition, general health and prior medical history of the patient being treated, and like factors well known in the medical arts.

In one embodiment, the therapeutically effective amount of zileuton is administered to a patient at a total daily dose from about 450 mg to about 2400 mg per day, from about 450 mg to about 1200 mg per day, from about 500 mg to about 1000 mg per day, or from about 600 mg to about 900 mg per day. In another embodiment, the therapeutically effective amount of zileuton is administered at a total daily dose of about 600 mg per day, about 700 mg per day, about 800 mg per day, about 900 mg per day, about 1000 mg per day, about 1200 mg per day, or about 2400 mg per day. In another embodiment, the zileuton is administered at about 600 mg twice a day (BID). In another embodiment, the zileuton is administered at about 1200 mg twice a day (BID).

In yet another embodiment, the zileuton is administered to the patient at about 1200 mg twice a day (BID) for a period of time sufficient to reduce the size of the nasal polyps, and administration of zileuton is continued after the reduction in size of the nasal polyps at about 600 mg twice a day (BID) for a period of time sufficient to maintain the reduction in size of the nasal polyps.

Alternatively, since it has recently been found that (R)-zileuton is more efficacious than either of (S)-zileuton and racemic zileuton in inhibiting 5-lipoxygenase activity, (R)-zileuton can be administered at a lower therapeutically effective dose. For example, a therapeutically effective amount of (R)-zileuton can be administered to a patient at a total daily dose from about 500 mg to about 1000 mg per day, from about 600 mg to about 900 mg per day, about 600 mg per day, about 900 mg per day, or about 1000 mg per day. The total daily dose of zileuton can be administered as a single dose or as multiple, divided doses. In one embodiment, the total daily dose is administered as a single daily dose. In another embodiment, the total daily dose is administered as two doses.

The route of administration of the zileuton and/or steroid depends on the condition to be treated. For example, oral administration may be preferred for treatment of severe NPs, and topical intranasal administration may be preferred to treat mild cases of NPs. The route of administration and the dosage, or amount, of the zileuton to be administered can be determined by the skilled artisan without undue experimentation in conjunction with standard dose-response studies. In one embodiment, the zileuton is administered with a pharmaceutically acceptable excipient. The excipient included with the zileuton of the invention is also chosen based on the expected route of administration of the zileuton in therapeutic applications.

The zileuton and/or steroid can be administered by a variety of routes including, but not limited to, nasal, topical, oral, pulmonary, parenteral, buccal, transdermal, intravenous, intramuscular, subcutaneous, and intradermal. In one embodiment the zileuton is administered orally. In another embodiment, the zileuton is administered topically. In another embodiment, the zileuton is administered nasally.

In one embodiment, the zileuton and/or steroid of the present invention is administered orally. For the purpose of oral therapeutic administration, the zileuton of the present invention may be incorporated with excipients and used in the form of tablets, troches, capsules, elixirs, suspensions, syrups, wafers, chewing gums and the like. Tablets, pills, capsules, troches and the like may also contain binders, excipients, disintegrating agent, lubricants, glidants, sweetening agents, and flavoring agents. Some examples of binders include microcrystalline cellulose, gum tragacanth or gelatin. Examples of excipients include starch or lactose. Some examples of disintegrating agents include alginic acid, corn starch and the like. Examples of lubricants include magnesium stearate or potassium stearate. An example of a glidant is colloidal silicon dioxide. Some examples of sweetening agents include sucrose, saccharin and the like. Examples of flavoring agents include peppermint, methyl salicylate, orange flavoring and the like. Materials used in preparing these various compositions should be pharmaceutically pure and non-toxic in the amounts used. In another embodiment, the zileuton is administered as a tablet or a capsule.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor, and the like.

As used herein, nasally administering or nasal administration includes administering the zileuton to the mucus membranes of the nasal passage or nasal cavity of the patient. As used herein, the zileuton is prepared by well-known methods to be administered, for example, as a nasal spray, nasal drop, suspension, gel, ointment, cream or powder. Administration of the zileuton may also take place using a nasal tampon or nasal sponge.

For topical administration, suitable formulations may include biocompatible oil, wax, gel, powder, polymer, or other liquid or solid carriers. Such formulations may be administered by applying directly to affected tissues, for example, a liquid formulation can be administered dropwise to the patient's nose, or a cream formulation can be administered to the nose.

The zileuton of the present invention can be administered parenterally such as, for example, by intravenous, intramuscular, intrathecal or subcutaneous injection. Parenteral administration can be accomplished by incorporating the zileuton of the present invention into a solution or suspension. Such solutions or suspensions may also include sterile diluents such as water for injection, saline solution, fixed oils, polyethylene glycols, glycerine, propylene glycol or other synthetic solvents. Parenteral formulations may also include antibacterial agents such as, for example, benzyl alcohol or methyl parabens, antioxidants such as, for example, ascorbic acid or sodium bisulfite and chelating agents such as EDTA. Buffers such as acetates, citrates or phosphates and agents for the adjustment of tonicity such as sodium chloride or dextrose may also be added. The parenteral preparation can be enclosed in ampules, disposable syringes or multiple dose vials made of glass or plastic.

Transdermal administration includes percutaneous absorption of the zileuton through the skin. Transdermal formulations include patches, ointments, creams, gels, salves and the like.

In addition to the usual meaning of administering the formulations described herein to any part, tissue or organ whose primary function is gas exchange with the external environment, for purposes of the present invention, "pulmonary" will also mean to include a tissue or cavity that is contingent to the respiratory tract, in particular, the sinuses. For pulmonary administration, an aerosol formulation containing the zileuton, a manual pump spray, nebulizer or pressurized metered-dose inhaler as well as dry powder formulations are contemplated. Suitable formulations of this type can also include other agents, such as antistatic agents, to maintain the disclosed compounds as effective aerosols.

A drug delivery device for delivering aerosols comprises a suitable aerosol canister with a metering valve containing a pharmaceutical aerosol formulation as described and an actuator housing adapted to hold the canister and allow for drug delivery. The canister in the drug delivery device has a head space representing greater than about 15% of the total volume of the canister. Often, the compound intended for pulmonary administration is dissolved, suspended or emulsified in a mixture of a solvent, surfactant and propellant. The mixture is maintained under pressure in a canister that has been sealed with a metering valve.

In addition to the common dosage forms set out above, the composition of the present invention may also be administered by controlled release means, delivery devices, or both, as are well known to those of ordinary skill in the art, such as those described in U.S. Patent Nos. 3,845,770; 3, 916, 899; 3, 536, 809; 3, 598,123; 4, 008, 719; 5,674,533; 5, 059, 595; 5, 591, 767; 5,120,548; 5,073,543; 5,639,476; 5,354,556; and 5,733,566. These pharmaceutical compositions can be used to provide slow or controlled-release of the active ingredient therein using, for example, hydropropylmethyl cellulose in varying proportions to provide the desired release profile, other polymer matrices, gels, permeable membranes, osmotic systems, multilayer coatings, microparticles, liposomes, microspheres, or the like, or a combination thereof. The controlled-release of zileuton may be stimulated by various inducers, for example pH, temperature, enzymes, water, or other physiological conditions or compounds. The term "controlled-release" in the context of the present invention is defined herein as the inclusion in the pharmaceutical composition of a compound or compounds, including polymers, polymer matrices, gels, permeable membranes, liposomes, microspheres, or the like, or a combination thereof, that facilitates the controlled-release of zileuton in the pharmaceutical composition.

### EXAMPLES

The present invention is further illustrated by the following examples, which should not be construed as further limiting. The contents of all figures and all references, patents and published patent applications cited throughout this application, as well as the Figures, are expressly incorporated herein by reference in their entirety.

### Example 1

A 16 year old female patient has a history of cystic fibrosis and chronic sinusitis. The patient complained of hyposmia and nasal congestion. Nasal endoscopy demonstrated bilateral nasal polyposis and purulence. Culture of the sinuses grew out *Pseudomonas.* Patient was prescribed ciprofloxacin per the sinus culture. Patient did not want to be on oral prednisone for the nasal polyps so zileuton was prescribed for the nasal polyposis. Prior to prescribing oral zileuton, a liver function test was performed that was normal. Patient was given zileuton 1200 mg BID. Two months later patient came back with improved nasal congestion and smell. Nasal endoscopy demonstrated no purulence and decreased polyps while on zileuton. Interestingly, the patient did not take the oral ciprofloxacin that was prescribed. Patient did have increased coughing with the zileuton 1200 mg BID, so the prescription was decreased to 600 mg BID. Since the patient's symptoms improved, the zileuton was eventually discontinued. After a few months the patient's sinus infection returned. Patient was placed on oral ciprofloxacin. Patient had initial improvement but eventually had return of her nasal symptoms, so the zileuton was restarted at 600 mg BID.

### Example 2

A 5 year old male had a history of cystic fibrosis and chronic sinusitis. Patient failed medical treatment, so he underwent endoscopic sinus surgery. Patient's sinus culture has grown MRSA and *Pseudomonas.* Patient initially did well after the surgery but was readmitted for pulmonary exacerbation. Despite aggressive medical treatment such as inhaled steroid and inhaled dornase alfa, the patient continued to have coughing. Patient was treated with ciprofloxacin for *Pseudomonas.* Patient's coughing initially improved but gradually returned. Nasal endoscopy was not performed in the child due to his young age. The patient was then treated with zileuton 600 mg BID after a normal liver function test. Patient's coughing improved with the zileuton treatment.

### Equivalents

Those skilled in the art will recognize, or be able to ascertain using no more that routine experimentation, many equivalents to the specific embodiments of the invention described herein. Such equivalents are intended to be encompassed by the following claims.

## Claims

1. Zileuton for use in the treatment of nasal polyps in a patient having cystic fibrosis.

2. The compound for use according to claim 1, wherein the patient has previously been treated with a steroid.

3. Zileuton for use in the treatment of nasal polyps in a patient having cystic fibrosis wherein the patient is also administered a steroid.

4. The compound for use according to claim 2 or 3, wherein the steroid is administered to the patient for a period of time sufficient to reduce the size of the nasal polyps by at least 50%.

5. The compound for use according to claim 4, wherein the administration of zileuton is begun after the 50% reduction in size of the nasal polyps.

6. The compound for use according to claim 4, wherein the administration of the steroid and zileuton is concurrent, and administration of zileuton is continued after the 50% reduction in size of the nasal polyps for a period of time sufficient to maintain the size reduction of the polyps.

7. The compound for use according to claim 2 or 3, wherein the steroid is administered for a period of time sufficient to reduce the size of the nasal polyps by at least 90%, and administration of zileuton is begun after the 90% reduction in size of the nasal polyps.

8. The compound for use according to claim 1, wherein zileuton is administered after the nasal polyps have been debrided.

9. The compound for use according to claim 8, wherein the patient is also administered a steroid.

10. The compound for use according to claim 8, wherein the zileuton is administered for a period of time sufficient to reduce the risk of recurrence of nasal polyps.

11. The compound for use according to any one of the proceeding claims, wherein zileuton is substantially free of (S)-zileuton.

12. The compound for use according to any one of claims 2-7 or 9, wherein the steroid is a steroid nasal spray, optionally wherein the steroid nasal spray is fluticasone, budesonide, flunisolide, mometasone, triamcinolone, or beclomethasone.

13. The compound for use according to any one of claims 2-7 or 9, wherein the steroid is an oral steroid.

14. The compound for use according to claim 13, when the oral steroid is prednisolone, prednisone, methylprednisolone or dexamethasone.

15. Zileuton for use in reducing the likelihood of developing nasal polyps in a patient having cystic fibrosis and a Pseudomonas infection.

16. The compound for use according to claim 15, comprising testing the patient for a Pseudomonas infection.

## Patentansprüche

1. Zileuton zur Verwendung bei der Behandlung von Nasenpolypen bei einem Patienten mit zystischer Fibrose.

2. Verbindung zur Verwendung gemäß Anspruch 1, wobei der Patient zuvor mit einem Steroid behandelt wurde.

3. Zileuton zur Verwendung bei der Behandlung von Nasenpolypen bei einem Patienten mit zystischer Fibrose, wobei dem Patienten außerdem ein Steroid verabreicht wird.

4. Verbindung zur Verwendung gemäß Anspruch 2 oder 3, wobei das Steroid dem Patienten über einen ausreichend langen Zeitraum verabreicht wird, um die Größe der Nasenpolypen um mindestens 50% zu reduzieren.

5. Verbindung zur Verwendung gemäß Anspruch 4, wobei mit der Verabreichung von Zileuton begonnen wird, nachdem die Größe der Nasenpolypen um 50% reduziert ist.

6. Verbindung zur Verwendung gemäß Anspruch 4, wobei das Steroid und Zileuton gleichzeitig verabreicht werden und die Verabreichung von Zileuton, nachdem die Größe der Nasenpolypen um 50% reduziert ist, über einen ausreichend langen Zeitraum fortgesetzt wird, um die Größenreduktion der Polypen aufrechtzuerhalten.

7. Verbindung zur Verwendung gemäß Anspruch 2 oder 3, wobei das Steroid über einen ausreichend langen Zeitraum verabreicht wird, um die Größe der Nasenpolypen um mindestens 90% zu reduzieren und mit der Verabreichung von Zileuton begonnen wird, nachdem die Größe der Nasenpolypen um 90% reduziert ist.

8. Verbindung zur Verwendung gemäß Anspruch 1, wobei Zileuton nach dem Débridement der Nasenpolypen verabreicht wird.

9. Verbindung zur Verwendung gemäß Anspruch 8, wobei dem Patienten außerdem ein Steroid verabreicht wird.

10. Verbindung zur Verwendung gemäß Anspruch 8, wobei das Zileuton über einen ausreichend langen Zeitraum verabreicht wird, um das Risiko des Wiederauftretens von Nasenpolypen zu reduzieren.

11. Verbindung zur Verwendung gemäß einem der vorhergehenden Ansprüche, wobei Zileuton im Wesentlichen frei von (S)-Zileuton ist.

12. Verbindung zur Verwendung gemäß einem der Ansprüche 2-7 oder 9, wobei das Steroid ein Steroid-Nasenspray ist, wobei das Steroid-Nasenspray optional Fluticason, Budesonid, Flunisolid, Mometason, Triamcinolon oder Beclometason ist.

13. Verbindung zur Verwendung gemäß einem der Ansprüche 2-7 oder 9, wobei das Steroid ein orales Steroid ist.

14. Verbindung zur Verwendung gemäß Anspruch 13, wenn das orale Steroid Prednisolon, Prednison, Methylprednisolon oder Dexamethason ist.

15. Zileuton zur Verwendung zum Reduzieren der Wahrscheinlichkeit, dass sich Nasenpolypen bei einem Patienten mit zystischer Fibrose und einer Pseudomonas-Infektion bilden.

16. Verbindung zur Verwendung gemäß Anspruch 15, die das Testen des Patienten auf eine Pseudomonas-Infektion beinhaltet.

## Revendications

1. Zileuton pour une utilisation dans le traitement de polypes du nez chez un patient atteint de mucoviscidose.

2. Composé pour une utilisation selon la revendication 1, dans laquelle le patient a reçu un traitement antérieur par un stéroïde.

3. Zileuton pour une utilisation dans le traitement de polypes du nez chez un patient atteint de mucoviscidose, dans laquelle le patient reçoit également un stéroïde.

4. Composé pour une utilisation selon la revendication 2 ou 3, dans laquelle le stéroïde est administré au patient pendant une période de temps suffisante pour produire une réduction d'au moins 50 % de la taille des polypes du nez.

5. Composé pour une utilisation selon la revendication 4, dans laquelle l'administration de zileuton est débutée après qu'une réduction de 50 % de la taille des polypes du nez a été obtenue.

6. Composé pour une utilisation selon la revendication 4, dans laquelle l'administration du stéroïde et de zileuton est simultanée et dans laquelle l'administration de zileuton est poursuivie après que la réduction de 50 % de la taille des polypes du nez a été obtenue pendant une période de temps suffisante pour maintenir la réduction de la taille des polypes.

7. Composé pour une utilisation selon la revendication 2 ou 3, dans laquelle le stéroïde est administré pendant une période de temps suffisante pour produire une réduction d'au moins 90 % de la taille des polypes du nez et dans laquelle l'administration de zileuton est débutée après qu'une réduction de 90 % de la taille des polypes du nez a été obtenue.

8. Composé pour une utilisation selon la revendication 1, dans laquelle le zileuton est administré après ablation des polypes du nez.

9. Composé pour une utilisation selon la revendication 8, dans laquelle le patient reçoit également un stéroïde.

10. Composé pour une utilisation selon la revendication 8, dans laquelle le zileuton est administré pendant une période de temps suffisante pour réduire le risque de récurrence des polypes du nez.

11. Composé pour une utilisation selon l'une quelconque des revendications précédentes, dans laquelle le zileuton est essentiellement exempt de (S)-zileuton.

12. Composé pour une utilisation selon l'une quelconque des revendications 2-7 ou 9, dans laquelle le stéroïde est administré en pulvérisations nasales et, en option, dans laquelle le stéroïde administré en pulvérisations nasales est le fluticasone, le budésonide, le flunisolide, le mométasone, le triamcinolone ou la béclométhasone.

13. Composé pour une utilisation selon l'une quelconque des revendications 2-7 ou 9, dans laquelle le stéroïde est administré par voie orale.

14. Composé selon la revendication 13, dans laquelle le stéroïde administré par voie orale est la prednisolone, la prednisone, la méthylprednisolone ou la dexaméthasone.

15. Zileuton pour une utilisation dans la réduction du risque de développement de polypes du nez chez un patient atteint de mucoviscidose et d'une infection à *Pseudomonas.*

16. Composé pour une utilisation selon la revendication 15, comprenant la recherche d'une infection à *Pseudomonas* chez le patient.
